# EUROPEAN PATENT APPLICATION

(11) **EP 0 530 490 A1**
(43) Date of publication of application: **10.03.1993**
(21) Application number: 92112791.6
(22) Date of filing: 27.07.1992
(51) Int. Cl.: G01N 33/50

(54) **A method and reagent composition for subtyping lymphocytes in peripheral blood**

(30) Priority: 07.08.1991 US 741374
(71) Applicant: MILES INC., Pittsburgh, PA 15219-2502 (US)
(72) Inventor: Kim, Young Ran, Hartsdale, New York 10530 (US); Paseltiner, Lynn, Monroe, New York 10950 (US); Yeh, Chien-kuo, Pleasantville, New York 10570 (US)
(74) Representative: Dänner, Klaus, Dr.

(57) **Abstract**

A rapid method for detection of monoclonal antibody labeled cells using forward light scatter/absorption clinical flow cytometers is disclosed. Calf-intestinal alkaline phosphatase is conjugated to mouse monoclonal antibodies (anti-CD2, CD3, CD4, CD8, CD19) for direct immunoenzymatic labeling. Red cells are pre-conditioned for lysis during the first incubation period with the alkaline phosphatase conjugated monoclonal antibodies by ammonium sulfate which is followed by an early fixation of white cells in the presence of dextrose. The combination of 5-bromo-4-chloro-3-indolyl phosphate and nitroblue-tetrazolium salt in diethanolamine buffer, containing zinc glycinate, at pH 9.5±0.1, is used as a buffer/substrate to yield stable, insoluble and intense purplish precipitates on the surface of the cells labeled with alkaline phosphatase conjugated monoclonal antibodies. The separation of granulocytes and lymphocytes is detected by a forward light scatter/absorption optics. Endogenous alkaline phosphatase in granulocytes is inhibited with levamisole. Early mild fixation of the white cells permits incubation at 35°C to 40°C which accelerates each step of the reaction without disrupting the cells throughout the procedures making the procedure adaptable for use on automated cytometric instrumentation.

## Description

### BACKGROUND OF THE INVENTION

### 1. Field of the Invention

The present invention relates to a method and reagent composition for immunophenotyping, and more particularly, a direct-labeling absorption-staining method and reagent therefor adapted for use in forward light scatter/absorption flow cytometers.

### 2. Description of the Prior Art

Flow cytometric analysis of lymphoid cells has had an important impact in many areas of immunological and biological research. A variety of monoclonal antibodies are available which bind specifically to various differentiation antigens expressed by cells of the immune system. Many such antibodies provide reliable reagents for enumeration of the lymphocyte subsets. Major applications are in monitoring the number of Helper T cells in peripheral blood from patients with acquired immunodeficiency syndrome (AIDS), and in characterizing the lineage of hemato-lymphoid malignancies and in transplantation medicine.

Most commonly, subsets of peripheral blood lymphocytes are identified by labeling the cells with fluorochrome - conjugated monoclonal antibodies (direct immunofluorescence) or with a fluorochrome - conjugated antibodies directed against unconjugated monoclonal antibodies (indirect immunofluorescence). The labeled cells, in a cell suspension, are identified by fluorescence and enumerated either microscopically or with a light scatter/fluorescence flow cytometer. See, for example, U.S. Patent No. 4,284,412. Typically, mononuclear cells separated in a gradient of Ficoll-Hypaque or leukocytes from a lysed whole blood sample are used for analysis.

Immunoenzymatic labeling using mouse monoclonal antibodies and anti-mouse IgG - immune complexes of alkaline phosphatase (AP) and monoclonal anti-AP (APAAP) has been found to give excellent immunocytochemical labeling of tissue sections and cell smears. Leukemic cells from myeloid and lymphocytic leukemias have been immunophenotyped by APAAP technique on blood and bone marrow smears, utilizing monoclonal antibodies reactive with myeloid or lymphoid antigens. Monoclonal antibody study of Philadelphia chromosome - positive blastic leukemias has also been performed on blood and bone marrow smears using APAAP technique. These APAAP procedures are, however, very long and tedious, and are not readily adaptable for use in automated methods. Conjugation of alkaline phosphatase directly to specific monoclonal antibodies would allow direct immunophenotyping of specific target cells by flow cytometry, shortening the procedure considerably, if a comparable sensitivity to that of APAAP technique could be obtained.

Alkaline phosphatase is a widely distributed family of isozymes found in a large number of species and tissues. Calf-intestinal alkaline phosphatase conjugates are a good choice for labeling to localize specific antigens because most endogenous tissue alkaline phosphatases can be specifically inhibited by levamisole which does not inhibit the intestinal form. A variety of substrates yielding colored products are used for the determination of alkaline phosphatase activity. P-nitrophenyl phosphate is frequently used to produce a soluble colored product, and naphthol AS-BI phosphate, naphthol-AS-MS phosphate are among the alkaline phosphatase substrates most frequently used with diazonium salts, to produce insoluble colored products. We investigated the above substrates for use in the present invention, but none generated adequately large absorption signals from the labeled cells for a clean separation. The combination of 5-bromo-4-chloro-3-indolyl phosphate (BCIP) and nitroblue-tetrazolium salt (NBT) was previously discovered by McGadey to produce enhanced sensitivity for the histochemical localization of alkaline phosphatase using the APAAP technique.

In view of the above drawbacks in the prior art, it became readily apparent that a rapid, direct, immuno-alkaline phosphatase method and associated reagents, developed for peripheral blood lymphocyte subtyping, which can be performed on a routine clinical hematology instrument such as the Technicon H^{.}1® blood analyzer (Technicon Instruments Corp., Tarrytown, New York) was needed.

### OBJECTS OF THE INVENTION

It is an object of the invention to provide a novel method and reagent composition useful for immunophenotyping involving the direct labeling absorption staining.

Another object of this invention is to provide a method and reagent composition as above which can be used for rapid, direct peripheral blood lymphocyte subtyping.

Yet another object of this invention is to provide a method and reagent composition as above which can be used in forward light scatter/absorption flow cytometers.

A still further object of this invention is to provide a method and reagent composition as above which can be performed at elevated temperatures, thus reducing the time required for such analysis as compared to existing techniques.

A yet further object of this invention is to provide a reagent composition in which unstained lymphocytes can be readily distinguished from unstained granulocytes by forward light scatter/cytometric techniques.

Still another object of the present invention is to provide a stable zinc containing alkaline solution.

A yet further object of the present invention is to provide a stable suspension of labeled and stained white blood cells.

Other objects and features of the present invention will be, in part, apparent, and, in part, pointed out hereinafter.

### SUMMARY OF THE INVENTION

The present invention provides a rapid, simple, accurate and sensitive method and reagent composition therefor for subtyping lymphocytes in peripheral blood utilizing alkaline phosphatase conjugated monoclonal antibody labeled cells employing forward light scatter/ absorption flow cytometric techniques.

In accordance with a preferred embodiment of the present invention, calf-intestinal alkaline phosphatase is conjugated to mouse monoclonal antibodies (anti-CD2, CD3, CD4, CD8, CD19) for directed immunoenzymatic labeling. The red cells are preconditioned for lysis during the first incubation period with the alkaline phosphatase conjugated monoclonal antibodies by ammonium sulfate which was followed by an early fixation of white cells in the presence of dextrose. The combination of 5-bromo-4-chloro-3-indolyl phosphate and nitroblue-tetrazolium salt in diethanolmaine buffer, containing zinc glycinate, is selected as buffer/substrate to yield stable, insoluble and intense purplish precipitates on the surface of the cells labeled with the monoclonal antibody-alkaline phosphatase conjugate, and a clean separation of granulocytes and lymphocytes as detected by a forward light scatter/absorption optics.

In accordance with another aspect of the invention, endogenous alkaline phosphatase in granulocytes is inhibited with levasimole. Early mild fixation of white cells permits incubation of cells at elevated temperatures, e.g. 38±1°C which accelerates each step of the reaction without disrupting the cells throughout the procedure. The method is competitive with direct immunofluorescence whole blood method used on fluorescence flow cytometers in speed, sensitivity and accuracy as demonstrated with AP conjugated anti-CD2, CD3, CD4, CD8 and CD19 Mabs.

### DESCRIPTION OF THE DRAWINGS

The above and other objects and significant advantages of the present invention are believed made clear by the following detailed description thereof taken in conjunction with the accompanying drawings wherein:
FIG. 1 is a series of cytograms demonstrating lymphocyte subsets determined in accordance with the present invention;
FIG. 2 is the EpicsPROFILE® linear green fluorescence pulse height distributions of unlabeled control cells and labeled with FITC - conjugated anti- CD2, CD3, CD4, CD8 and CD19;
FIGs. 3a-3d represent cytograms of a normal whole blood samples. FIG. 3a represents unlabeled sample processed without levamisole; FIG. 3b unlabeled sample processed with levamisole; FIG. 3c sample labeled with alkaline phosphatase conjugated anti-monocyte processed with levamisole; and Fig. 3d sample labeled with alkaline phosphatase conjugated anti-CD4 processed with levamisole;
FIG. 4 is a correlation plot of the lymphocyte subsets determined by prior art immunofluorescence method, and by the present invention.
FIG. 5 are cytograms of anti-CD4-AP labeled cells demonstrating another aspect of the present invention;
FIG. 6 illustrates the effect of zinc concentration on the scatter/absorption signal of granulcotyes/labeled and stained lymphocytes;
FIG. 7 are cytograms of unlabeled lymphocytes and granulocytes demonstrating another aspect of the present invention;
FIG. 8 is a correlation of results obtained from freshly prepared cells and results obtained post-24 hours practicing the present invention; and
FIG. 9 are cytograms of anti- CD₄-AP labeled cells obtained from freshly prepared cells and post-24 hours practicing the present invention.

### DESCRIPTION OF THE PREFERRED EMBODIMENT

A significant feature of the present invention is the use of alkaline-phosphatase conjugated monoclonal antibodies to perform immunophenotyping by a direct labeling-absorption staining technique in a forward light scatter/absorption flow cytometer.

For accurate counting of lymphocyte subsets by the proposed method, it is essential that a clean separation of lymphocyte cluster from all other clusters of cells be obtained. A clean separation of unstained lymphocytes from unstained granulocytes along the forward-angle light scatter axis is a very difficult task. Scatter-fluorescence flow cytometers such as Coulter's Epics systems or B-D's Facs systems separate the two cell clusters using two axes, forward angle and right angle light scatter. The Technicon H^{.}1® or Technicon H^{.}2® instrument has only one light scatter channel, forward scatter, in the peroxidase optics which is used for lymphocyte typing.

We discovered that adding a small amount of zinc, such as zinc chloride, zinc sulfate or zinc acetate at concentrations of from about .07 to about 0.25 mM to the buffer/substrate increases the pulse height of forward angle light scatter signals generated by granulocytes, presumably because zinc prevents loss of granulocyte proteins.

We also discovered that dextrose in the fixative at a concentration of from about 7% to about 15% (v/v) and NBT at a concentration of from about 0.5 mM to about 2.0 mM in the dye/substrate/buffer (in addition to the zinc) made it possible to separate granulocytes from unstained lymphocytes.

Complete lysis of red cells in whole blood samples in the shortest possible time is essential in developing a rapid method for peripheral blood lymphocyte subtyping. Incompletely lysed red cell ghosts are a source of undesirable origin noise. We further discovered that adding a small amount of ammonium sulfate to the diluent of alkaline phosphatase conjugated monoclonal antibodies and incubating whole blood with the mixture for a short period of time pre-conditions the red cells for later rapid lysis through the subsequent fixation step. Therefore, in accordance with another aspect of the present invention, ammonium sulfate at a concentration of from about 1% to about 5%, preferably 2.5% (v/v), is added to the solution containing alkaline phosphatase conjugated monoclonal antibodies (20 µl of, this solution is added to 100 µl of whole blood for labeling) during the first 5 minutes incubation at a temperature of from about 35°C to about 40°C and preferably 38±1°C to achieve two purposes: (i) antibody binding to target cells; and (ii) pre-conditioning of red cells.

In method development, obtaining accurate and reproducible data is a very important factor. Since substrate turnover by alkaline phosphatase is continuous, it was very difficult to fix the position of absorption signals generated by alkaline phosphatase active cells (labeled lymphocytes, and eventually granulocytes even in the presence of levamisole). The staining of the prepared cell suspensions kept increasing with time. Stopping alkaline phosphatase activity by acidifying the cell suspension created many problems such as lowering the granulocyte scatter signals. Adding formaldehyde alone to the cell suspension, pH 9.5±0.1, shifted forward angle scatter signals of all cell clusters higher, thereby making the analysis of the sample after a defined incubation time period (about three hours) no longer possible. Adding known inhibitors of calf-intestinal alkaline phosphatase such as EDTA did not stabilize the height of absorption signals.

In accordance with another aspect of the present invention, a combination of formaldehyde at a concentration of about 2% to about 6% (v/v) and phosphate buffer (25 mM to 100 mM) at almost neutral pH, 6.0-7.0 is used to stop the staining increase of the prepared cell suspension for an extended period of time, e.g. one week, without deteriorating the integrity of the cytogram of samples. This permits analysis of prepared cell suspension at any convenient time within a week of preparation which generates accurate and reproducible results.

### EXAMPLES

The following examples set forth the method and reagent composition of our invention. Standard commercially available reagent grade materials were used whenever possible. It will be understood that the reagents and the procedures which follow are provided for purpose of illustration only, and that other ingredients, proportions and procedures can be employed in accordance with the disclosures of this invention.

### Materials and Methods

Calf-intestinal alkaline phosphatase (AP) (Biozyme, San Diego, CA) was conjugated to mouse monoclonal antibodies, anti-CD2, CD3, CD4, CD8 and CD19 (all from Technicon Instruments Corp., Tarrytown, NY) using homo- and hetero-bifunctional cross-linking reagents, 2-immunothiolane (2-IT) and N-hydroxy-succinimide ester of N-(4-carboxy-cyclohexyl-methyl) maleimide (SMCC) (both from pierce, Rockford, IL). An anti-monocyte-AP conjugate was also prepared using anti-M77.7 monoclonal antibody (Kim Y.R., Abraham N.C., Lutton J.D., "Mechanisms of Differentiation of U937 Leukemic Cells Induced by GM-CSF and 1,25(OH₂-Vitamin D₃", Leukemia Res., 1991, 15: 409-418) to investigate the location of the monocyte population in the H^{.}1 cytogram. The conjugation method was modified from the method of Yoshitake, et al., 1982; Lambert, et al., 1983 (Lambert J.M., Jue R., Traut R.R., "Disulfide Cross-Linking of Escherichia Coli Ribosomal Proteins With 2-iminothiolane (methyl 4-mercaptobutyrimidate): Evidence that the Cross-Linked Protein Pairs are Formed in the Intact Ribosomal Subunit", Biochemistry, 1978, 17: 54006-5416; and Yoshitake S, Imakawa M., Ishikawa E., et al., "Mild and Efficient Conjugation of Rabbit Fab' and Horseradish Peroxidase Using a Maleimide Compound and Its Use for Enzyme Immunoassay", J. Biochem., 1982, 92: 1413-1424).

The monoclonal antibodies and AP molecules were separately activated. First, the monoclonal antibodies were reacted with 2-IT to introduce spacer "arms" with -SH group on the monoclonal antibody molecules. The AP was reacted with SMCC to introduce maleimide groups on the AP molecules. The coupling reaction was then carried out by mixing the two activated proteins in a triethanolamine buffer 100 mmol/L containing sodium chloride 100 mmol/L and tetra-sodium EDTA 1 mmol/L (pH 7.3) to produce the antibody-AP conjugates. Maleimide reacts specifically with -SH group producing a stable linkage, a "flexible extended arm", between the two protein molecules. The antibody-AP conjugates were isolated from free monoclonal antibody and AP molecules by eluting through a Sephacryl-300 column (Pharmacia, Piscataway, NJ) with a tris buffer 50 mmol/L containing sodium chloride 100 mmol/L, magnesium acetate 2 mmol/L, zinc sulfate 1 mmol/L and disodium EDTA 1 mmol/L (pH 7.3). The isolated conjugates were then diluted with a tris buffer containing protease free bovine serum albumin 2 g/L, ammonium sulfate 189 mmol/L and sodium azide 1 g/L to a final IgG concentration (10 mg/L for CD3, 15 mg/L for CD4 and 25 mg/L for CD2, CD8 and CD19, respectively).

The following reagents were used to prepare samples for lymphocyte typing:
a) alkaline phosphatase conjugated monoclonal antibodies (anti-CD₂, CD₃, CD₄, CD₈ and CD₁₉) in a solution containing tris buffer 50 mM, protease free BSA 2 g/L, ammonium sulfate 25 g/L, and sodium azide 1 g/L;
b) a fixative containing dextrose 555 mmol/L and HCHO 1.67 mmol/L in phosphate buffer 75 mmol/L (pH 7.0);
c) a lysing reagent containing NH₄Cl 155 mmol/L, KHCO₃ 10 mmol/L, Na₄EDTA 1.2 mmol/L and formaldehyde 25 mmol/L;
d) a buffer/substrate solution containing NBT (Sigma, St. Louis, MO) 1 mmol/L and BCIP (JBL, San Luis Obispo, CA) 1.2 mmol/L, sodium chloride 100 mmol/L, magnesium acetate 1 mmol/L, and zinc glycinate 0.10 mmol/L in diethanolamine buffer 80 mmol/L (pH 9.5±0.1);
e) a cell washing solution (CWS) containing protease free bovine serum albumin (Miles, Kankakee, IL) 2 g/L, and levamisole (L[-]-2,3,5,6-tetra-hydro-6-phenyl-imidazol[2,1-b]thiazole) 3 mM/L, Kathon CG 0.03% (pH 7.4) in a hepes buffer 10 mmol/L (pH 7.4);
f) a propylene glycol (PG) solution containing PG 600 g/L, formaldehyde 533 mmol/L in phosphate buffer 75 mmol/L (pH 6.5).

EDTA-anticoagulated whole blood specimens from thirty-one (31) presumably normal donors were processed as follows: 100 µl of an EDTA - anticoagulated whole blood specimen was mixed with 20 µl of a monoclonal antibody solution, and was incubated at a temperature of preferably 38±1°C in a heating block for 5 min. (All incubations were carried out in the heating block.) Then, 200 µl of the fixative was added, mixed and incubated for an additional 5 min. After which, 2 ml of the lysing reagent was added, mixed and incubated for an additional 5 min. to lyse red cells. The remaining white cells were then washed twice with CWS by centrifuging in a Serofuge™ for 1 min., decanting the supernatant, dislodging and resuspending the cell button each time. Then, 0.5 ml of the buffer/substrate solution was added, mixed and incubated for 5 min. to stain the labeled cells. Finally, 0.5 ml of the PG solution was added and mixed (to match the refractive index of the cell suspension to that of the H^{.}1 peroxidase channel flow cell sheath fluid). Formaldehyde and phosphate in the PG solution (pH 6.5) are used to terminate AP activity in the final cell suspension.

The prepared samples were analyzed on a Technicon H^{.}1® hematology analyzer (Technicon Instruments Corporation, Tarrytown, NY) using its direct cytometry port. The H^{.}1 peroxidase channel is equipped with forward light scatter/absorption optics. A software program was incorporated into the H^{.}1 instrument software system to automatically display, threshold, calculate and print out the percent-labeled lymphocytes of each sample (Technicon H^{.}1® System Operator's Guide, Tarrytown, NY, Technicon Instruments Corp., 1990). No hardware modification of the instrument was necessary to perform the test. Granulocytes and monocytes are ignored in this calculation. An Epics®PROFILE (Coulter, Hialeah, FL) flow cytometer was used as a reference instrument for a fluorescence method (Training Guide for the Epics®PROFILE, Coulter Educational Center, Miami Lakes, FL, Coulter Instrument Corp., 1987). The reference monoclonal antibodies used for Epics®PROFILE analysis were FITC - conjugated OKT3(CD3), OKT4(CD4), OKT8(CD8), OKT11(CD2) (all from Ortho, Raritan, NJ) and Leu12(CD19) (Becton Dickinson, Mountain View, CA). The samples were prepared according to each manufacturers' protocol. For both the H^{.}1® and Epics®PROFILE methods, two readings were taken from each preparation, and an average of the replicate readings was used to present the results.

### Results

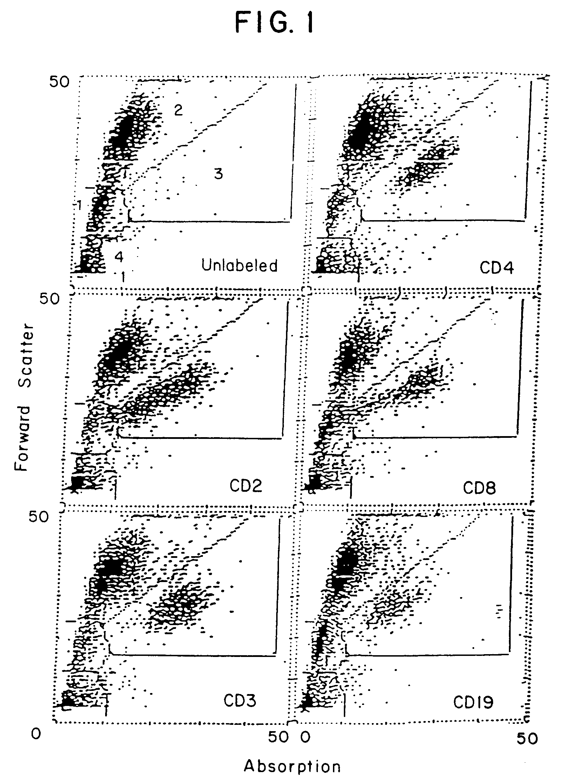

Lymphocyte subsets determined with the immuno-alkaline phosphatase method of the present invention, analyzed on a Technicon H^{.}1® are illustrated in the cytograms of FIG. 1. The ordinates represent forward light scatter signal height, and abscissas represent absorption signal heights. The cells in threshold box 1, 2, 3 and 4 represent unlabeled lymphocytes, unlabeled granulocytes (neutrophils, eosinophils and monocytes), labeled lymphocytes and origin noise (platelets and red cell ghosts) respectively. Results for unlabeled control cells and cells labeled with alkaline phosphatase conjugated anti- CD2 (Pan T cells, E-rosette positive), CD3 (Pan T cells, mitogenic), CD4 (Helper T cells), CD8 (Suppressor T cells) and CD 19 (B cells).

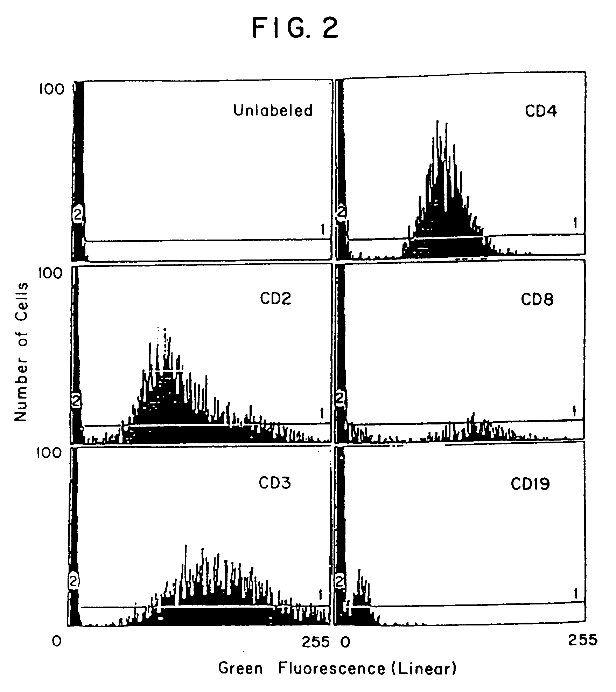

EpicsPROFILE® linear green fluorescence pulse height distributions for corresponding samples of unlabeled control cells and labeled with FITC-conjugated anti- CD2, CD3, CD4, CD8 and CD19 are presented in FIG. 2, and show a similar variability in the density distributions of cell surface antigens among the lymphocytes of each of the substrates. The lymphocyte cluster is gated on the cytogram of forward angle light scatter vs. right angle light scatter and reanalysed in the forward angle light scatter vs. green fluorescence channel.

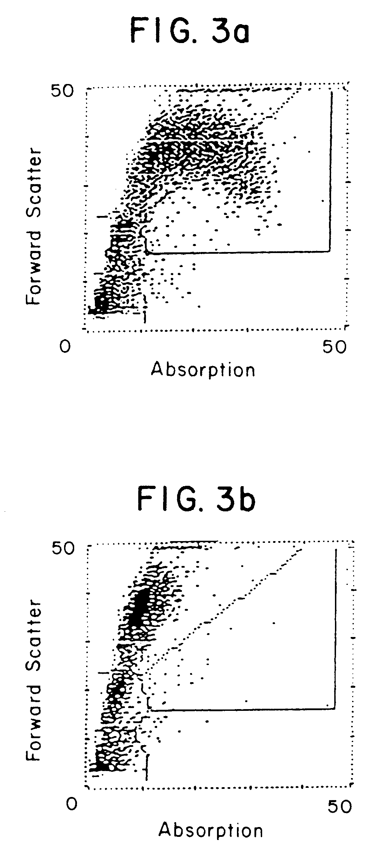

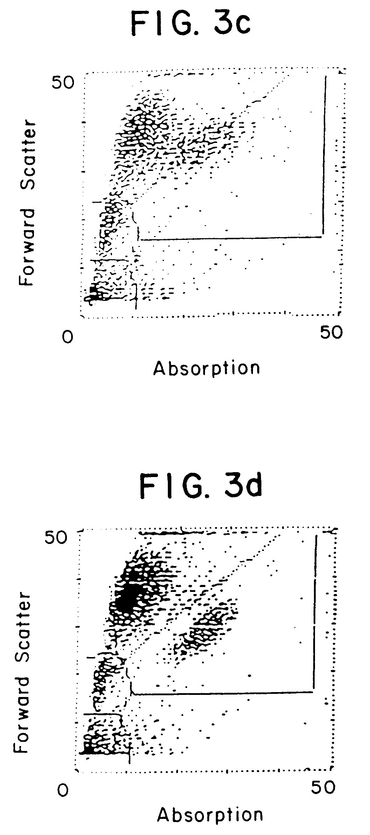

Light-microscopic examination of the alkaline phosphatase-stained suspensions of cells revealed that labeled lymphocytes were stained dark-purple; unlabeled lymphocytes were unstained; endogenous alkaline phosphatase in granulocytes was selectively inhibited by the addition of levamisole, and therefore, was not stained; red cell ghosts were not visible; platelets were not stained. H^{.}1 cytograms of unlabeled cell suspension processed with and without levamisole, anti-monocyte-AP labeled cell suspension processed with levamisole, and anti-CD4-AP labeled cell suspension processed with levamisole are shown in FIG. 3. The granulocytes in the cell suspension, processed without levamisole, were stained due to their endogenous alkaline phosphatase activity. As can be seen in FIG. 3a, the stained granulocyte population shifted to the right falling beyond the labeled lymphocyte threshold into the positive lymphocyte (LPOS) box. When the same sample was processed with levamisole, however, the granulocytes were no longer stained, and consequently, the absorption signals from the granulocytes remained in the large unstained cell (LUC) threshold box (FIG. 3b).

To investigate the location of the monocyte population in the H^{.}1 cytogram of this method, the same sample was labeled with anti-monocyte-AP conjugate and processed in the presence of levamisole. As demonstrated in FIG. 3c, the absorption signals of the labeled and stained monocytes traverse across the absorption threshold into the LPOS box from the LUC box, indicating that unlabeled and unstained monocyte signals in this method are normally within the LUC box along with granulocytes (neutrophils and eosinophils). Since many monocytes are known to express a low level of CD4 molecules on their cell surface, the same sample was labeled with anti-CD4-AP conjugate, stained, and the changes in the granulocyte distribution in the H^{.}1 cytogram were carefully examined (FIG. 3d). A slightly bulging cluster of the granulocytes (probably because of the stained CD4⁺ monocytes), can be observed. However, the signals from the bulge did not enter the LPOS box, therefore, they did not interfere with CD4⁺ lymphocyte count.

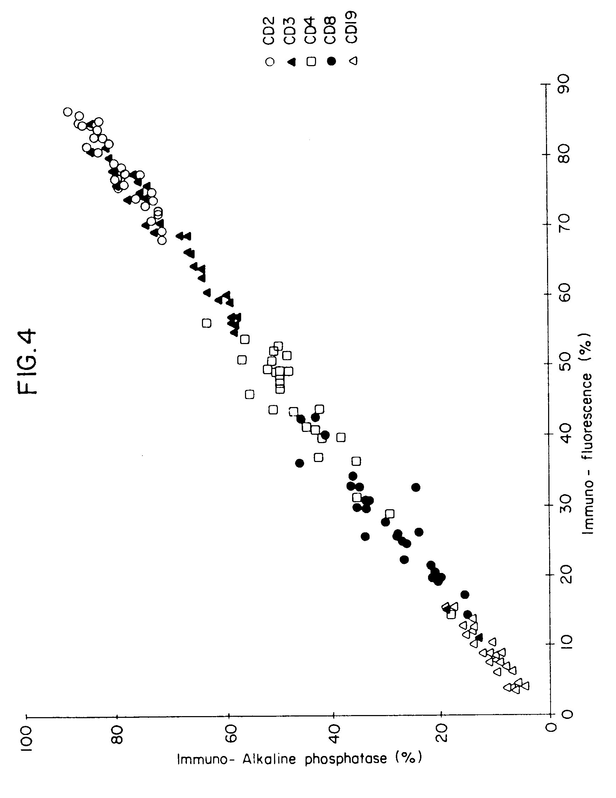

Linear regression plots of the results by the immunofluorescence method (EpicsPROFILE data) vs. those by the present method for all lymphocyte subsets (CD2, CD3, CD4, CD8 and CD19) are shown in FIG. 4. The correlation data presented in Table 1 (below) show good agreement between the two methods for each lymphocyte subset; the data show insignificant slope or intercept bias for all the subsets except for CD2⁺ Pan T cells.

**TABLE 1**

| Correlation Between Results by EpicsPROFILE (x) vs. H^{.}1 (y) | | | | | | |
|---|---|---|---|---|---|---|
| Parameter (CD#) | % labeled x mean | % labeled y mean | r^{a} | slope | y-intercent | S_{yx} ^{b} |
| Pan T (CD2) | 80.1 | 79.2 | 0.95 | 0.88 | 8.65 | 1.59 |
| Pan T (CD3) | 72.2 | 71.8 | 0.98 | 0.95 | 3.21 | 1.61 |
| Helper T (CD4) | 46.0 | 47.4 | 0.95 | 0.96 | 3.23 | 3.02 |
| Suppressor T (CD8) | 29.2 | 30.4 | 0.97 | 1.02 | 2.98 | 2.98 |
| Pan B (CD19) | 9.4 | 11.4 | 0.94 | 0.99 | 2.10 | 1.27 |

| | | | | | | |
|---|---|---|---|---|---|---|
| ^{a} Correlation coefficient | | | | | | |
| ^{b} Standard error of estimate Number of donor samples assayed = 31 each | | | | | | |

The imprecision of the present method presented in Table 2 (below) is computed from the duplicate readings of each of the same 31 samples used for the correlation study. Ninety-five percent confidence intervals show less than two units variation for all subsets.

**TABLE 2**

| Imprecision of H^{.}1 Lymphocyte Typing Method | | | | |
|---|---|---|---|---|
| Parameter (CD#) | Mean (%) | SD (%) | 95% CI (%) | Range of Samples Tested (%) |
| Pan T (CD2) | 79.2 | 0.56 | 1.20 | 71.1 - 88.0 |
| Pan T (CD3) | 71.8 | 0.92 | 1.20 | 58.3 - 84.1 |
| Helper T (CD4) | 47.4 | 1.01 | 1.30 | 18.0 - 62.9 |
| Suppressor T (CD8) | 30.4 | 1.26 | 1.60 | 14.9 - 73.1 |
| Pan B (CD19) | 11.4 | 0.63 | 0.82 | 4.3 - 18.8 |

During the development of this whole blood immunopheno-typing method by direct AP labeling, we experienced many problems in obtaining a cytogram that cleanly and reproducibly separate granulocytes from unlabeled lymphocytes, granulocytes from labeled lymphocytes, and labeled lymphocytes from unlabeled lymphocytes using a forward light scatter/absorption flow cytometer. There were many reasons for these problems; granulocytes were stained because of their endogenous alkaline phosphatase; the monoclonal antibody-AP conjugates were either unstable or may have produced steric hindrance near the monoclonal antibody-binding sites; forward angle light scatter signals from unstained granulocytes were too close to that of large unlabeled lymphocytes,

All the problems stated above were resolved by selecting and optimizing the combination of reagent composition and assay conditions: A possible steric hindrance near the monoclonal antibody-binding sites by the large alkaline phosphatase molecules of the conjugates was resolved by creating a stable "long-arm" between the antibody molecule and the alkaline phosphatase molecule, using SMCC and 2-IT as cross-linking agents. The prepared monoclonal antibody-AP conjugates demonstrated over a year stability under refrigerated conditions (see the monoclonal antibody-diluent composition in Materials).

The combination of dextrose in the fixative, NBT, BCIP, zinc glycinate in the buffer, and formaldehyde in the PG solution at the concentrations stated herein helped to produce sufficiently large scatter signals from the granulocytes to separate them cleanly from the unlabeled lymphocytes along the forward angle light scatter axis, This combination of reagents prevented loss of granulocyte proteins throughout the procedure and increased the refractive index of the granulocytes, thus increasing the pulse height of forward angle light scatter signals from the cells.

We discovered that adding a small amount of zinc such as, but not limited to, zinc chloride, zinc sulfate and zinc acetate at concentrations of from about .07 to 0.25 mM to the buffer/substrate increases the pulse height of forward angle light scatter signals generated by granulocytes, presumably because zinc prevents loss of granulocyte proteins.

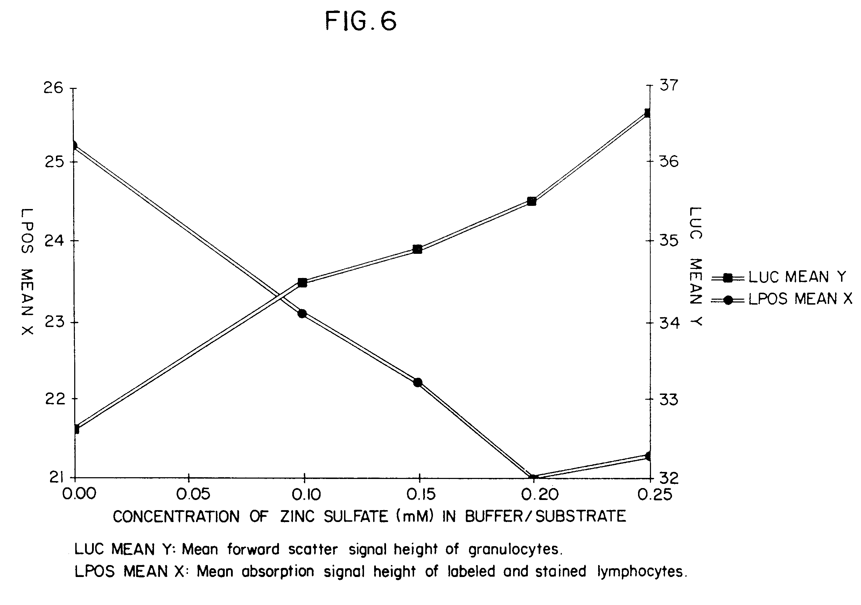

Zinc forms insoluble precipitates of zinc hydroxide at alkaline pH. To keep zinc in solution in this DEA buffer, pH 9.5±0.1, we searched for a suitable zinc chelating agent. Most common metal chelating agents such as EDTA or hydroxy-EDTA inhibited AP activity, and excised the effect of Zinc on granulocytes, deteriorating the separation of granulocytes from unstained lymphocytes. Addition of glycine or citrate prevented zinc precipitation in the buffer for an extended period of time without decreasing the forward scatter signals generated by granulocytes. In addition, zinc glycinate or zinc citrate minimized the inhibition effect of zinc itself on the catalytic activity of AP (contrary to literature findings, zinc in the buffer inhibited AP activity at 0.1 mM or above). The height of scatter signals from granulocytes (LUC Mean Y), and the height of absorption signals from the labeled and stained lymphocytes (LPOS Mean X) are illustrated in FIG. 6 and are expressed as mean channel numbers of H^{.}1 scatter and absorption signals. As the concentration of zinc (zinc sulfate used) was increased in the buffer/substrate, LUC Mean Y was increased in a linear fashion while LPOS Mean X was decreased.

Levamisole in the CWS inhibited endogenous AP activity in the granulocytes, maintaining a clean separation of granulocytes from labeled lymphocytes (see FIG. 3). Ammonium sulfate in the monoclonal antibody-AP diluent "pre-conditioned" red cells for later rapid and complete lysis of red cells, even in the presence of the fixative. The early mild fixation of the white cells prevented capping of the cell surface antigen molecules, and also permitted incubation at 38 ± 1°C which accelerated each step of the reaction without degrading the quality of cytograms of the samples.

Alternatively, the levamisole may be added to the buffer/substrate, however, we noticed that when this is done, the levamisole precipitates from solution over time at the alkaline pH of the buffer, thereby negatively affecting the stability of the buffer solution.

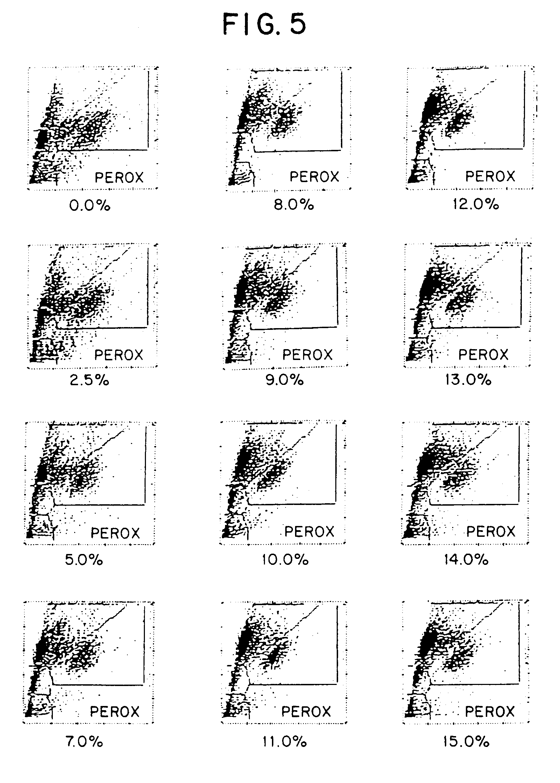

The effects of dextrose concentration in the fixative will be appreciated from FIG. 5 which illustrates cytograms of anti-CD4-AP labeled cells. The concentration of dextrose in the fixative was varied, as labeled, while keeping the formaldehyde concentration constant at 4%. At 0% dextrose concentration, almost all of the granulocyte signals are in the unlabeled lymphocyte box, except monocytes which are in the labeled lymphocyte box. Monocytes express some CD4 molecules, and therefore, labeled with anti-CD4-AP monoclonal antibody in this preparation. As the concentration of dextrose was increased, the scatter signals of all granulocytes were increased, thereby separating granulocytes from lymphocytes.

Formaldehyde and phosphate in the PG solution (pH 6.5) inhibited further AP reaction in the cell suspension without altering the cytograms. This permitted analysis of prepared cell suspension at any convenient time within a week of preparation while generating accurate and reproducible results.

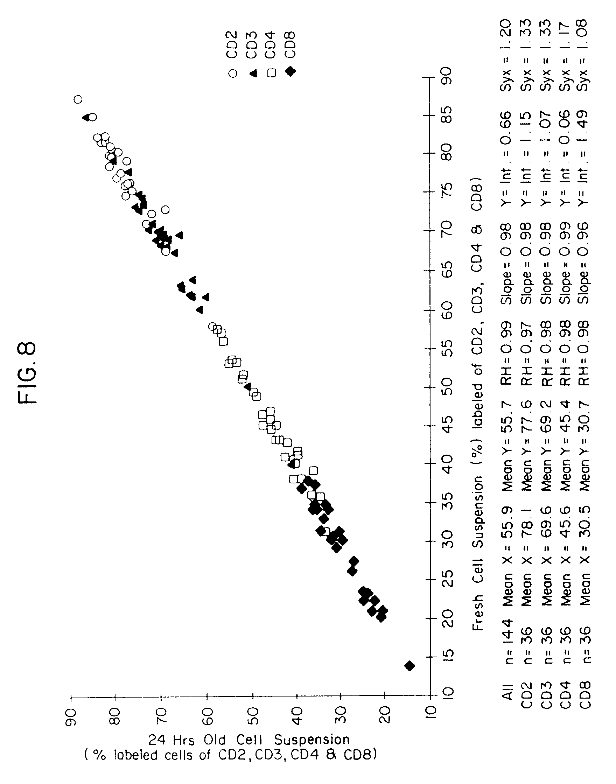

A comparison of the results obtained when the subject invention is practiced using a freshly prepared cell suspension and the results obtained from post-24 hour cell suspension are presented in FIG. 8, and illustrate the stability of the cell suspension over time.

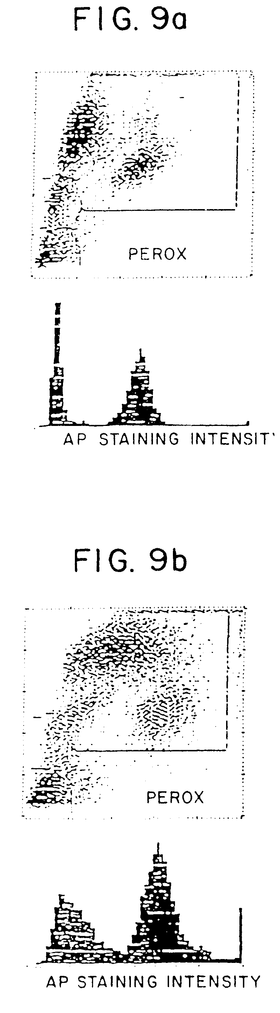

The stability of the cell suspension is further illustrated in FIG. 9. FIG. 9a is a cytogram obtained on a freshly prepared cell suspension of anti-CD4-AP labeled cells. The labeled CD4+ lymphocytes of this sample was 55.1%. FIG. 9b is a cytogram obtained on the same cell preparation, except that the reading was taken 24 hours later. The cell suspension was stored for 24 hours without the stabilizing agent (HCHO and phosphate in 60% PG) under refrigeration. Note that the percentage of CD4+ cells was increased from 55.1% to 87.9%, due primarily to the invading absorption signals of stained granulocytes.

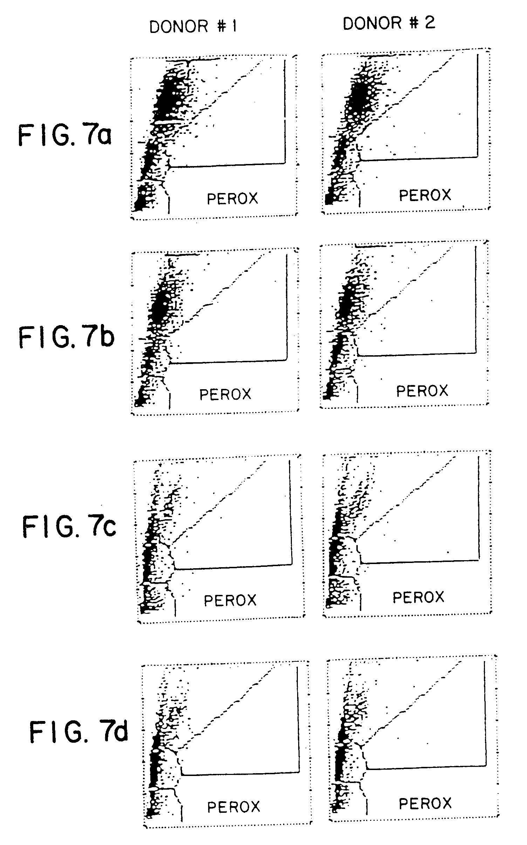

The positive effects of NBT and BCIP in the buffer/substrate solution can best be appreciated with reference to FIG. 7. In FIG. 7a, the control buffer/substrate has both NBT and BCIP in DEA buffer as described in the method discussed hereinabove. FIG. 7b illustrates the resulting cytogram produced when BCIP was left out of the buffer/substrate formulation. In FIG. 7c, NBT was left out of the buffer/substrate formulation. In FIG. 7d, both NBT and BCIP were left out from the formulation. As can be seen in these figures, NBT plays an important role in separating granulocytes from lymphocytes. Dextrose in the fixative alone does not do the job. Note that 1.0 mM of zinc is in the buffer/substrate as described in the Materials section. Without zinc in the buffer, LUC Mean Y will be much lower, even in the presence of NBT.

As can be seen in FIG. 3C, monocyte - signals in this preparation fall with granulocytes, and therefore, do not interfere with labeled lymphocyte counts. Absolute positive lymphocyte counts for each subset can be obtained, if desired, by multiplying the percent labeled cells of the sample with the absolute lymphocyte counts of the corresponding sample (either manually or automatically via a modified software), run in the CBC/DIFF mode of the same instrument (H^{.}1® or H^{.}2®).

It should be noted that other lymphocyte subsets besides the five presented here should be capable of analysis using the disclosed method. It is also possible to detect subsets of other blood and bone marrow cells using similar techniques.

The method described hereinabove was performed at elevated temperatures, i.e. at between about 35°C to about 40°C. It will be appreciated that it can be performed at lower temperatures; however, the time necessary to complete the various steps in the procedure may have to be extended appropriately in order to allow the reactions to be completed.

It will also be appreciated that the risk of HIV virus infection will be substantially eliminated due to the exposure of the whole blood sample to formaldehyde during the procedure.

The method presented here is competitive with direct immunofluorescence whole blood methods used on fluorescence flow cytometers in speed, sensitivity and accuracy.

Some of the advantages of the present invention evident from the foregoing description include an improved procedure for lymphocyte subtyping utilizing scatter/absorption techniques, and a reagent composition therefor, which are adapted for use on an automated flow cytometer.

In view of the above, it will be seen that the various objects of our invention are achieved and other advantageous results attained.

The various features and advantages of the invention are thought to be clear from the foregoing description. However, various other features and advantages not specifically enumerated will undoubtedly occur to those versed in the art, as likewise will many variations and modifications of the preferred embodiments illustrated, all of which may be achieved without departing from the spirit and scope of the invention as defined by the following claims.

## Claims

1. An automated method for identifying and enumerating cells of a select subclass of lymphocytes in blood comprising the steps of:
a) providing an aliquot of whole blood;
b) incubating said aliquot with a reagent composition comprising:
i) antibodies which are selectively reactive with distinct antigenic determinants of the surface of cells in said subclass, said antibodies conjugated to alkaline phosphatase to label the cells in said subclass; and
ii) ammonium sulfate to pre-condition the red cells for lysis;
c) fixing the white blood cells by adding a fixative reagent comprising dextrose and formaldehyde;
d) lysing the red blood cells by adding a lysing reagent comprising ammonium chloride;
e) washing said fixed white cells with a cell washing solution comprising an inhibiting reagent to inhibit the activity of endogenous alkaline phosphatase;
f) staining said fixed and inhibited white cells in a second reagent composition comprising a buffer, a chromogen and a substrate for alkaline phosphatase;
g) inhibiting further catalytic activity of the alkaline phosphatase by adding a stabilizing reagent comprising formaldehyde, phosphate buffer and propylene glycol;
h) passing the cells through an electro-optical detection system adapted to detect the light scattered and absorbed by said cells; and
i) differentiating cells of said subclass based at least in part on said light scattered and absorbed by said cells.

2. The method of Claim 1 wherein said incubating step is conducted at a temperature of from about 35°C to about 40°C.

3. The method of any of Claims 1 and 2 wherein said concentration of ammonium sulfate in said reagent composition is from about 1% to about 5% (v/v).

4. The method of any of Claims 1 to 3 wherein the concentration of dextrose in said fixative is from about 7% to about 15% (v/v).

5. The method of any of Claims 1 to 4 wherein the concentration of formaldehyde in said fixative is from about 3% to about 6% (v/v).

6. The method of any of Claims 1 to 5 wherein said inhibiting reagent includes levamisole.

7. The method of any of Claims 1 to 6 wherein said second reagent composition further includes zinc.

8. The method of Claim 7 wherein the concentration of zinc is from about 0.7 mM to about 0.25 mM.

9. The method of any of Claims 1 to 8 wherein said second reagent composition further includes a zinc chelating agent.

10. The method of any of Claims 1 to 9 wherein the concentration of formaldehyde in said stabilizing reagent is from about 2% to about 6% (v/v).
